# EUROPEAN PATENT APPLICATION

(11) **EP 2 752 242 A1**
(43) Date of publication of application: **09.07.2014**
(21) Application number: 11871623.2
(22) Date of filing: 02.09.2011
(51) Int. Cl.: B01J 29/40, C07C 1/00, C07C 1/04, C07C 15/00, C07C 2/00

(54) **METHOD FOR OBTAINING HYDROCARBONS FROM LOWER ALCOHOLS**

(71) Applicant: Guradoor, S.L., 38390 Santa Úrsula (Tenerife) (ES)
(72) Inventor: GONZÁLEZ GONZÁLEZ, Daniel, 38390 Santa Ursula (Tenerife) (ES)
(74) Representative: Gil-Vega, Victor
(86) International application number: PCT/ES2011/070618
(87) International publication number: WO 2013/030415

(57) **Abstract**

The invention relates to a method for the production of energy from lower alcohols, particularly methanol, wherein such alcohols are obtained from synthesis gas by means of a process comprising the gasification-pyrolysis of wet crushed coal. According to the method of the invention, the aforementioned alcohols are subject to a catalytic dehydration using a zeolite catalyst as an acid catalyst, giving rise to olefins which in turn, using the same catalyst as a molecular sieve, give rise to highly branched paraffins and cyclic and aromatic compounds, by means of hydrogenation, alkylation and isomerisation, using the hydrogen present in the above-mentioned synthesis gas.

## Description

The present invention relates to a method for the production of energy from lower alcohols, particularly methanol, wherein such alcohols are obtained through a process involving the gasification-pyrolysis of wet crushed coal in the presence of different catalysts.

Specifically, the synthesis gas obtained from the coal gasification-pyrolysis process is fed to a catalytic reactor, for instance, of the Lurgi type, for the obtaining of methanol at a temperature ranging between 240-270°C.

CO + H₂ →CH₃OH ΔH < 0

CO₂ + H₂ →CH₃OH ΔH < 0

The alcohol thus obtained is used, for instance, in the production of industrial and domestic fuels.

Methanol is being increasingly used worldwide for a series of innovative applications aimed at meeting a growing energy demand. Methanol is a clean alternative energy which can be obtained from natural gas, coal and different renewable resources, such as biomass, landfill biogases or the emissions of power and industrial plants. The characteristics of methanol as a liquid fuel at room temperature and the different sources used to produce it make it a good fuel alternative for motor cars, trucks and buses.

Specifically, methanol is used as one of the components of the biodiesel production process, as well as a raw material for the production of Dimethyl ether, a clean combustion liquid gas mainly used for cooking and heating, and which is also a good substitute of diesel fuel. It is also used for commercial purposes, in innovative technologies for the transformation of Methanol into olefins and Methanol into gasoline.

The reaction of methanol to obtain hydrocarbons was discovered at the end of the seventies decade by the research team of the company Mobil. This reaction must be catalysed with zeolite-type catalysts and especially, the zeolite ZSM-5. The reaction allows, through the intermediary of two consecutive dehydrations, the conversion of Methanol into dimethyl ether and the subsequent transformation of this latter product into hydrocarbons, initially, light olefins. The reaction is a refining process which includes a broad range of products, from methane to durene. Depending on the reaction variables (temperature, spatial velocity, partial pressure of supply, acidity of the catalyst...) the selectivity of the reaction to transform Methanol into hydrocarbons may be modified, giving rise to different sub-processes, such as MTG (Methanol to gasolines) or MTO (Methanol to olefins) among others.

The main problem posed by the Methanol to hydrocarbons reactions is the deactivation of the zeolitic catalysts as a result of the generation of deactivating coke inside the pores of the catalyst. This process may even entail the clogging of the pores and deactivate the active centres of the catalyst, decreasing, and even cancelling its activity.

Similarly to the alkenes obtained from the cracking, essentially acetylene, the alkenes obtained as waste products from the process aimed at obtaining synthesis gas may be converted through hydration into alcohols of interest from a synthetic point of view. Since ethylene and water react during the gaseous stage (vapour) and the reaction is developed in that direction, decreasing the number of molecules present in the mixture, it stems that the displacing of the equilibrium in the direction of the alcohol formation contributes to the pressure increase. In this case, in order to achieve that the reaction is carried out at sufficient speed, the use of the catalyst and the heating of the substances are required. However, as the reaction is of an exothermic nature, an excessively strong heating will accelerate the reaction, which is developed with heat absorption, or in other words, the decomposition of the alcohol formed and the displacement of the equilibrium on the opposite direction. It has been established that the optimum conditions for ethylene hydration are a temperature range of 280 to 300°C and a pressure range of 7 to 8 Mpa, using the phosphoric acid deposited on a solid carrier as reaction catalyst. Under these conditions, around 5% of the initial ethene is transformed into alcohol upon its passing through the contact device. Consequently, to yield the reaction profitable, it is necessary to separate the alcohol from the reaction products and recirculate the ethene for a new hydration, i.e., the circulation process must be implemented. It is also evident that the exhaust products of the reaction may be used to heat the substances arriving for hydration.

On the other hand, the dehydration of the alcohols obtained requires the presence of an acid and heat. Generally speaking, two different methods may be employed: (a) heating the alcohol with sulphuric or phosphoric acid, and (b) passing the vapour through a catalyst, preferably alumina (Al₂O₃), at high temperatures (Alumina works as an acid, as a Lewis acid or, through the intermediary of OH groups in its surface, as a Lowry-Bronsted acid).

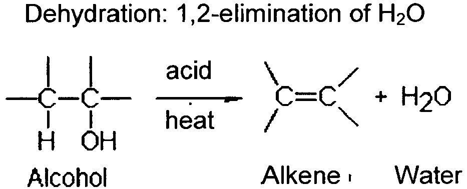

As opposed to the base-induced 1,2-elimination, dehydration is a reversible process.

Similarly, the hydrogenation of the residual olefin products resulting from the obtaining of synthesis gas through a coal gasification-pyrolysis process leads to the obtaining of alkanes, that will be subsequently subject to reforming and isomerization processes to obtain branched-chain alkanes with a high energy content or aromatic compounds.

Thus, the object of the present invention is to provide a procedure for the production of energy from lower alcohols, particularly methanol, wherein such alcohols are derived from a synthesis gas obtained through a process involving the gasification-pyrolysis of coal, which allows the conversion of such alcohols into olefins, after their dehydration with zeolites, such olefins being in turn transformed into highly branched paraffins and cyclical and aromatic compounds.

To that effect, the Methanol obtained from the synthesis gas, as it has been previously explained, is passed trough a zeolitic catalyst at a temperature ranging from 340°C to 375°C, and this temperature is reached through heating with circulating water obtained from the gasification-pyrolysis device.

The catalytic properties of zeolites are the direct consequence of their high surface area and types of active centres. The dehydration, or elimination of water from an alcohol molecule leads to the formation of alkenes or olefins. This elimination reaction requires an acid catalyst, which is used to protonate the hydroxyl group and convert it into a proper leaving group through the formation of a carbonyl ion and, consequently, the reactivity depends on the easiness to form such ion. In some cases, a protonated alcohol may be attacked by another alcohol molecule. This reaction occurs when the dehydration takes place in primary non impaired alcohols, and the result of the process is the formation of water and one ether.

Generally speaking, the acidity of a zeolite depends on the Al atoms present at its crystalline network. However, not all the acid centres of zeolite show the same degree of activity and, therefore, not all of them are able to catalyse these dehydrogenation reactions. For that purpose, the zeolitic catalyst used in the process according to the invention may be optionally activated, preferably with ammonium or nitric acid. For instance, the values of the conversion percentages for 1-pentanol reveal that the activated zeolite is an excellent catalyst for the dehydration of linear alcohols, since they show a high degree of activity in the conversion of 1-pentanol, without a specific order of catalyst activity, and the conversion ranges between 99 and 100%. However, it changes in the case of the conversion of Isopropyl alcohol (branched alcohol). The most probable explanation for this behaviour is reflected on the CIC, since CIC decreases when zeolite is activated with acid and, therefore, the number of cations present at the structure and available for their exchange gradually decreases. This implies that, since there are less exchangeable cations, the space between them must be larger, and the final result is the presence of larger pores, close to the meso- region. The opposite procedure (activation with ammonium nitrate) entails a greater CIC (a larger number of exchangeable ions) and a greater number of cations is incorporated into the structure of zeolite, and the pores of the structure are smaller, located in the micro- region, which entails a limitation for this catalyst prepared to be used in reactions involving a voluminous substrate. The products obtained in the different reactions were 1-pentene and isopropene, respectively.

On the other hand, the alkenes obtained in these alcohol dehydration reactions are converted into the relevant alkanes through hydrogenation, wherein the required hydrogen is derived from the synthesis gas obtained during the gasification-pyrolysis process, and in turn, these alkanes are converted into other branched alkanes with a higher octane rating by means of isomerization and reforming processes, through the relevant hydrogenation, isomerization and reforming processes carried out within the relevant reactors.

Thus, as it has been previously mentioned, and according to the method of the invention, the methanol obtained from the synthesis gas derived from a coal gasification-pyrolysis process is passed through an optionally activated zeolitic catalyst, at a temperature ranging from 340°C and 375°C, such temperature being reached by heating it with the circulating steam obtained from the gasification-pyrolysis device.

At this point the dehydration of the alcohol occurs through the active acid centres of the zeolite catalyst, and the result will be a mixture of olefins which, in turn, are partly converted into paraffins by the same catalyst, by means of an alkylation process, and the catalyst operates as a molecular sieve which allows to separate the molecules obtained on the basis of their pore size.

The mixture which now comprises different molecular species is passed through a fractional distillation column to separate them, resulting in alkane fractions of a linear and branched type, as well as aromatic compounds and, to a lesser extent, residual alkenes.

The method according to the invention allows to eliminate the main disadvantage of the reaction of Methanol with hydrocarbons, namely, the deactivation of the zeolitic catalysts through the formation of deactivating coke inside the pores of the catalyst, since the processing temperature and the recirculation of the residual hydrogen and water towards the gasification-pyrolysis reaction do not allow the deposition of C, neither on the surface nor on the pores of the catalyst.

## Claims

1. Method for the production of energy from lower alcohols, particularly methanol, wherein such alcohols are obtained from synthesis gas by means of a process involving the gasification-pyrolysis of wet crushed coal, **characterized in that** the aforementioned alcohols are subject to a catalytic dehydration using a zeolite catalyst as an acid catalyst, giving rise to olefins which in turn, using the same catalyst as a molecular sieve, give rise through hydrogenation, alkylation and isomerisation, to highly branched paraffins and cyclic and aromatic compounds, using the hydrogen present in the above-mentioned synthesis gas.

2. Method for the production of energy from lower alcohols according to claim 1, **characterized in that** the methanol obtained from the synthesis gas derived from a coal gasification-pyrolysis processed is passed through an optionally activated zeolitic catalyst, at a temperature ranging from 340°C to 375°C, and this temperature is reached through heating with circulating steam from the gasification-pyrolysis device.

3. Method for the production of energy from lower alcohols according to claim 1, **characterized in that** the mixture resulting from the process, which consists of different molecular species, is passed through a fractional distillation column to separate them, resulting in alkane fractions of a linear and branched type, as well as aromatic compounds and, to a lesser extent, residual alkenes.
